# EUROPEAN PATENT APPLICATION

(11) **EP 1 870 093 A1**
(43) Date of publication of application: **26.12.2007**
(21) Application number: 06115677.4
(22) Date of filing: 19.06.2006
(51) Int. Cl.: A61K 31/00, A61K 45/06, A61P 13/12

(54) **Pharmaceutical combination comprising adenosine A1 receptor antagonists and radiocontrast media**

(71) Applicant: Solvay Pharmaceuticals GmbH, 30173 Hannover (DE)
(72) Inventor: Hocker, Berthold, 30173 Hannover (DE); Fischer, Yvan, 30890 Barsinghausen (DE); Witte, Klaus, 30173 Hannover (DE); Ziegler, Dieter, 30966 Hemmingen (DE)
(74) Representative: Gosmann, Martin

(57) **Abstract**

The present invention relates to pharmaceutical combinations comprising a therapeutically effective amount of at least one selective adenosine A1 antagonist combined with at least one radiocontrast media. The invention also relates to the use of said combinations in the manufacture of a medicament for the treatment of radiocontrast media induced nephropathy. Furthermore, the invention is relating to a kit comprising a single dosage form of said combination of at least one adenosine A1 antagonist and at least one radiocontrast media.

## Description

### FIELD OF INVENTION

The present invention relates to pharmaceutical combinations comprising a therapeutically effective amount of at least one selective adenosine A1 receptor antagonist and at least one radiocontrast media (RM). The invention also relates to the use of said combinations in the manufacture of a medicament for the treatment of radiocontrast media induced nephropathy. Furthermore, the invention relates to a kit comprising said combinations.

### BACKGROUND OF THE INVENTION

Interventional techniques, fast multislice computer tomographies and new 3D reconstruction techniques have increased the use of iodinated intravascular radiocontrast media (RM) over the last decades. The majority of examinations require iodinated RM for accurate and safe diagnosis and interventional procedures. Today approximately 60 million dosages are applied every year world wide (Andrew, 2004¹). Radiocontrast media can lead to a decline of excretory renal function that starts soon after administration. The renal dysfunction can be transient, persistent or even irreversible. Hence, the use of radiocontrast media has been associated with increased in-hospital morbidity, mortality, and cost of medical care and long admissions, especially in patients requiring dialysis. Radiocontrast media induced nephropathy (CIN) is therefore a clinically important problem. The definition of CIN varies. It can be defined as acute aggravation of renal functionality after application of RM, induced as proximate cause to the exclusion of alternative etiologies. The most common definition of a minor effect is an increase in serum creatinine greater than 25% or 44 mol/l (0.5 mg/dl) after the intravascular administration of a RM. A major effect is defined as increase in serum creatinine greater than 50% or 88 mmol/l (1 mg/dl). The pathogenesis of CIN is not fully understood. It is believed, that two main factors, hemodynamic as well as tubular effects, are involved. Application of RM leads to a change in renal hemodynamics, above all to a decrease in the glomerular filtration rate (GFR). GFR is the rate of ultra filtration of plasma across the walls of the glomerular capillaries and measurement of total GFR of both kidneys provides a sensitive index of overall renal excretory function. The glomerular filtration rate is calculated by comparing urine creatinine levels with the blood test results. A GFR value (see http://www.fpnotebook.com²) in a range of 97-137 ml/min/1.73 m² is adequate for a male human and of 88-128 ml/min/1.73 m is adequate for a female human, whereas a GFR lower than 15 ml/min/1.73 m² leads to kidney failure. A decrease in GFR induced by application of RM is considered to be the main cause for the development of CIN. Along the renal tubular system, substances like RM that are not reabsorbed become increasingly concentrated. Up to 99% of renal fluids are usually taken up by the action of manifold cellular and paracellular mechanisms. This means that the urine concentration of RM can increase by a factor of 100. Along with the continuous concentration process, tubular fluid containing RM will become increasingly viscous and can lead to tubular obstruction (Ueda, 1993³). Inevitably, intrarenal pressure increases as well, since the kidney cannot expand due to the surrounding capsule. As a consequence, renal perfusion pressure for the renal medulla may no longer be sufficient to warrant sufficient perfusion. In the kidney, activation of A1AR in afferent glomerular arterioles has been suggested to contribute to tubuloglomerular feedback (TGF), which is a strategic feedback mechanism, designed to control tubular flow and regional perfusion. The vasoconstriction elicited by elevations in [NaCl] in the macula densa region of the nephron. A role of adenosine in TGF response mediation is consistent with its effect to cause vasoconstriction. In addition to its vasoconstrictor effect, A₁ receptor stimulation contracts mesangial cells in the glomerulus (Olivera, 1989⁴). Acute renal failure caused by the injection of RM has been recognized for many years as a complication in diagnostic and interventional procedures. The incidence of acute renal failure directly induced by RM lies at approximately 10-15%, while the incidence of CIN defined by clinically significant increases in serum creatinine is as high as 22% (Porter, 1989⁵). The peak creatinine concentration occurs within 3-5 days of exposure to the contrast media and usually resolves satisfactorily, but in up to 10% of at risk patients, dialysis is required. Preexisting renal insufficiency reduced intravascular volume and additional underlying diseases (e.g. hypertension, diabetes mellitus) are said to be some of the leading risk factors for radiocontrast media induced nephropathy. The osmolality, the measurement of the number of molecules and particles in a solution per kilogram of water, of the RM is regarded to be of great importance in radiocontrast induced nephropathy. The incidence of nephropathy induced by low-osmolar RM is low in the general population and has been calculated to be less than 2 % (Nikolsky, 2003⁶).

The adenosine production is one of the discussed mechanisms behind CIN. Adenosine is an endogenous neuromodulator with predominantly inhibitory effects on the CNS, heart, kidneys and other organs. It is a naturally occurring nucleoside, which exerts its biological effects by interacting with a family of adenosine receptors known as A1, A2a, A2b, and A3, all of which modulate important physiological processes. Selective A1 adenosine receptor antagonists (A₁AR) have pronounced effects on the kidney, and have shown to be potent diuretics and natriuretics with little effect on potassium excretion. Thus, they are renal protective, useful for the treatment of renal failure, renal dysfunction, nephritis, hypertension, and edema. The kidney produces adenosine constitutively to regulate glomerular filtration and electrolyte reabsorption mediated by the adenosine A1-receptor system. The A1 adenosine receptor has been found to govern the vasoconstriction response of the afferent renal arteriole. Adenosine causes a reduction in the blood flow to the kidney, and thus a reduction in the glomerular filtration rate and the renal blood flow. Inhibition of the A1 receptor will heighten the glomerular filtration rate, and correspondingly increase the rate of urine formation. The application of adenosine receptor antagonists has been implicated in protection from acute renal failure. The adenosine receptor antagonists aminophylline (combination of theophylline and ethylenediamine 2:1) and theophylline (which has been found to non-selectively antagonize adenosine receptors in the brain) were evaluated as potential agents to protect against radiocontrast media induced nephropathy (Shammas, 2001; Welch, 2002; Huber, 2002⁷). Aminophylline does not appear to add a protective role in preventing radiocontrast media induced nephropathy while theophylline was effective in preventing radiocontrast media induced nephropathy impaired renal excretory, endocrine and tubular function. These results suggest that adenosine may play a role in the pathogenesis of CIN and that application of non-selective adenosine receptor antagonists has been implicated in protection from acute renal failure associated with RM treatment. Erley (1994⁸) investigated the influence of the non-selective adenosine antagonist theophylline on the glomerular filtration rate after the application of RM and allocates adenosine a major role in CIN. Furthermore, Arakawa (1996⁹) describe the role of adenosine in the renal responses to the contrast medium iohexol in dogs with and without pre-existing renal insufficiency. Arakawa indicates that in normal renal function, iohexol elicits renal vasodilation by activating mainly the adenosine A2 receptors. Whereas in impaired renal function, iohexol induces both A2 and A1 activation. Arakawa proposes the adenosine A2 receptors to be associated with the initial renal vasodilation, and the adenosine A1 receptors to be responsible for the sustained aggravation of renal hemodynamics. Yao (2000¹⁰) investigated the influence of the selective adenosine A1 antagonist KW-3902 on radiocontrast media induced nephropathy in rats with chronic nitric oxide deficiency. Yao suggested adenosine influencing the pathogenesis of CIN via the activation of the A1 receptors. Greiner (2005¹¹) studied the influence on theophylline and acetylcystein separately and in combination on radiocontrast media induced nephropathy in intensive care patients and corroborates the prophylactic properties of theophylline in CIN. Lee (2006¹²) concludes that renal A1 adenosine receptors are only partially responsible in the pathogenesis of radiocontrast nephropathy. In experiments with renal A1 adenosine receptors knockout mice was found, that these mice are protected from acute renal failure induced by RM injection. Direct tubular toxicity seemed, however, not to be modulated by renal A1 adenosine receptors. Patent application EP 1 386 609 (CV Therapeutics¹³) describes methods for restoring diuretic and renal function comprising adenosine A1 antagonist in combination with a diuretic. Patent application WO 99/31101 (Univ. South Florida¹⁴) discloses xanthine derivatives as adenosine A1 receptor antagonists. Further on, radiolabelled derivatives and a method of imaging the adenosine A1 receptor antagonists for medical diagnostic purposes are mentioned.

### SUMMARY OF THE INVENTION

A₁AR have been previously shown to possess protective effects in several nephrotoxic models of acute renal failure. Increased release of renal adenosine and stimulation of renal adenosine receptors have been proposed to be among the major reasons in development of radiocontrast media induced acute renal failure. We now surprisingly found that administration of selective A1 receptor antagonists, reduces the risk of CIN development.

It is therefore an object of the present invention to use a therapeutically effective amount of at least one selective adenosine A1 receptor antagonist for the manufacture of a medicament for the treatment of nephropathy induced by at least one radiocontrast media, for the increase in serum creatinine levels as well as for the decrease in renal blood flow, in mammals and humans.

A further object of the invention is pertaining to pharmaceutical combination comprising a therapeutically effective amount of at least one selective adenosine A1 receptor antagonist and a radiocontrast media.

A further object of the invention is pertaining to a kit comprising a therapeutically effective amount of at least one selective adenosine A1 receptor antagonist and a radiocontrast media.

The at least one A₁AR antagonist which can be used according to the present invention may be selected from the group of XAC, DPCPX, CPX, NAX, ENX, CVT-124, PACPX, BW-A844U, BW-A 522, SPT, KW-3902, KF15372, KFM 19, MDL 102,503, N-0861, N-0840, FK-352, FK-838, FK-453, WRC-0571, WRC-0342, WRC-0006, WRC-0007, DTI-0017, FR-166124, IRFI-165, GP-04012, EPI-2010, BW-1205U90, 4-[2-phenyl-7H-pyrrolo[2,3-d]-pyrimidin-4-ylamino]-trans-cyclohexanol, 4S-4-hydroxy-1-2-phenyl-7H-pyrrolo[2,3-d]pyrimidin-4-yl-L-prolinamide, N⁶-Butyl-8-phenyladenine, N⁶-Butyl-8-phenyladenine, 8-Phenyltheophylline, theophylline, aminophylline, midaxifylline, apaxifylline, naxifylline, Adentri, and/or a pharmaceutically acceptable salt, and/or a prodrug, and/or a solvate thereof.

The at least one RM which can be used according to the present invention may be an iodinated or gadolinium-based radiocontrast media selected from the group consisting of bunaiod, biligram, bilimiro, bilopaque, cholimil, ethiodol, diatrast, dionosil, falignost, gadobutrol, gadodiamide, gadopentetate dimeglumine, gastrografin, hexabrix, hippodin, mangafodipir, amidotrizoate, ethiodized oil, imagopaque, iodamide, iodipamide, iodixanol, iodophene, iophendylate, iomeron, iomeprol, iopamidol, iopanoic acid, iopiperidol, iophendylate, iopromide, iopydol, iosimenol, iothalamic acid, iotrolan, ioversol, ioxilan, ioxaglic acid, isopaque, ipodate, meglumine iothalamate, meglumine acetrizoate, meglumine diatrizoate, metrizamide, myelotrast, omnipaque, osbil, optiray, optojod, opacoron, perflutren, phenobutiodil, phentetiothalein sodium, priodax, propyliodone, skiodan, sodium iodomethamate, sodium diatrizoate, telepaque, teridax, tetrabrom, thorotrast, triognost, 1,3,5-Tri-n-hexyl-2,4,6-triiodobenzene, tyropanoate, visipaque or xenetix, and/or a pharmaceutically acceptable salt, and/or a prodrug, and/or a solvate thereof.

### DETAILED DESCRIPTION OF THE INVENTION

The present invention pertains to the use of a therapeutically effective amount of at least one selective adenosine A1 antagonist for the manufacture of a medicament for the prevention of nephropathy induced by at least one radiocontrast media in mammals or humans. The invention pertains thus to the use of a therapeutically effective amount of at least one selective adenosine A1 antagonist selected from the group of XAC, DPCPX, CPX, NAX, ENX, CVT-124, PACPX, BW-A844U, BW-A 522, SPT, KW-3902, KF15372, KFM 19, MDL 102,503, N-0861, N-0840, FK-352, FK-838, FK-453, WRC-0571, WRC-0342, WRC-0006, WRC-0007, DTI-0017, FR-166124, IRFI-165, GP-04012, EPI-2010, BW-1205U90, 4-[2-phenyl-7H-pyrrolo[2,3-d]-pyrimidin-4-ylamino]-trans-cyclohexanol, 4S-4-hydroxy-1-2-phenyl-7H-pyrrolo[2,3-d]-pyrimidin-4-yl-L-prolinamide, N⁶-Butyl-8-phenyladenine, N⁶-Butyl-8-phenyladenine, 8-Phenyltheophylline, theophylline, aminophylline, midaxifylline, apaxifylline, naxifylline, Adentri, and/or a pharmaceutically acceptable salt, and/or a prodrug, and/or a solvate thereof.

Furthermore, the invention relates to the use of a therapeutically effective amount of at least one selective adenosine A1 antagonist for the manufacture of a medicament for the prevention of increase in serum creatinine levels induced by at least one radiocontrast media in mammals or humans. The invention pertains thus to the use of a therapeutically effective amount of at least one selective adenosine A1 antagonist selected from the group of XAC, DPCPX, CPX, NAX, ENX, CVT-124, PACPX, BW-A844U, BW-A 522, SPT, KW-3902, KF15372, KFM 19, MDL 102,503, N-0861, N-0840, FK-352, FK-838, FK-453, WRC-0571, WRC-0342, WRC-0006, WRC-0007, DTI-0017, FR-166124, IRFI-165, GP-04012, EPI-2010, BW-1205U90, 4-[2-phenyl-7H-pyrrolo[2,3-d]-pyrimidin-4-ylamino]-trans-cyclohexanol, 4S-4-hydroxy-1-2-phenyl-7H-pyrrolo[2,3-d]pyrimidin-4-yl-L-prolinamide, N⁶-Butyl-8-phenyladenine, N⁶-Butyl-8-phenyladenine, 8-Phenyltheophylline, theophylline, aminophylline, midaxifylline, apaxifylline, naxifylline, Adentri, and/or a pharmaceutically acceptable salt, and/or a prodrug, and/or a solvate thereof.

Furthermore, the invention relates to the use of a therapeutically effective amount of at least one selective adenosine A1 antagonist for the manufacture of a medicament for the prevention of decrease in renal blood flow induced by at least one radiocontrast media. The invention pertains thus to the use of a therapeutically effective amount of at least one selective adenosine A1 antagonist selected from the group of XAC, DPCPX, CPX, NAX, ENX, CVT-124, PACPX, BW-A844U, BW-A 522, SPT, KW-3902, KF15372, KFM 19, MDL 102,503, N-0861, N-0840, FK-352, FK-838, FK-453, WRC-0571, WRC-0342, WRC-0006, WRC-0007, DTI-0017, FR-166124, IRFI-165, GP-04012, EPI-2010, BW-1205U90, 4-[2-phenyl-7H-pyrrolo[2,3-d]-pyrimidin-4-ylamino]-trans-cyclohexanol, 4S-4-hydroxy-1-2-phenyl-7H-pyrrolo[2,3-d]-pyrimidin-4-yl-L-prolinamide, N⁶-Butyl-8-phenyladenine, N⁶-Butyl-8-phenyladenine, 8-Phenyltheophylline, theophylline, aminophylline, midaxifylline, apaxifylline, naxifylline, Adentri, and/or a pharmaceutically acceptable salt, and/or a prodrug, and/or a solvate thereof.

Furthermore, the invention relates to the use of a therapeutically effective amount of at least one selective adenosine A1 antagonist for the manufacture of a medicament for the prevention of a risk or need of dialysis in a human or mammalian patient, preferably of transient, persistent or irreversible dialysis, said patient being subject to receive radiocontrast media. The invention pertains thus to the use of a therapeutically effective amount of at least one selective adenosine A1 antagonist selected from the group of XAC, DPCPX, CPX, NAX, ENX, CVT-124, PACPX, BW-A844U, BW-A 522, SPT, KW-3902, KF15372, KFM 19, MDL 102,503, N-0861, N-0840, FK-352, FK-838, FK-453, WRC-0571, WRC-0342, WRC-0006, WRC-0007, DTI-0017, FR-166124, IRFI-165, GP-04012, EPI-2010, BW-1205U90, 4-[2-phenyl-7H-pyrrolo[2,3-d]-pyrimidin-4-ylamino]-trans-cyclohexanol, 4S-4-hydroxy-1-2-phenyl-7H-pyrrolo[2,3-d]-pyrimidin-4-yl-L-prolinamide, N⁶-Butyl-8-phenyladenine, N⁶-Butyl-8-phenyladenine, 8-Phenyltheophylline, theophylline, aminophylline, midaxifylline, apaxifylline, naxifylline, Adentri, and/or a pharmaceutically acceptable salt, and/or a prodrug, and/or a solvate thereof.

The present invention further relates to a pharmaceutical combination of a therapeutically effective amount of at least one selective adenosine A1 antagonist, and at least one radiocontrast media, wherein the pharmaceutical combination being suitable for simultaneous, separate or step-wise administration to humans or mammals.

Furthermore, the present invention relates to a kit comprising a therapeutically effective amount of at least one selective adenosine A1 antagonist, and at least one radiocontrast media, wherein the pharmaceutical combination being suitable for simultaneous, separate or step-wise administration to humans or mammals.

The A1AR which can be used according to the present invention may be selected from the group of XAC, DPCPX, CPX, NAX, ENX, CVT-124, PACPX, BW-A844U, BW-A 522, SPT, KW-3902, KF15372, KFM 19, MDL 102,503, N-0861, N-0840, FK-352, FK-838, FK-453, WRC-0571, WRC-0342, WRC-0006, WRC-0007, DTI-0017, FR-166124, IRFI-165, GP-04012, EPI-2010, BW-1205U90, 4-[2-phenyl-7H-pyrrolo[2,3-d]-pyrimidin-4-ylamino]-trans-cyclohexanol, 4S-4-hydroxy-1-2-phenyl-7H-pyrrolo[2,3-d]pyrimidin-4-yl-L-prolinamide, N⁶-Butyl-8-phenyladenine, N⁶-Butyl-8-phenyladenine, 8-Phenyltheophylline, theophylline, aminophylline, midaxifylline, apaxifylline, naxifylline, Adentri, and/or a pharmaceutically acceptable salt, and/or a prodrug, and/or a solvate thereof..

The RM which can be used according to the present invention may be an iodinated or gadolinium-based radiocontrast media selected from the group consisting of bunaiod, biligram, bilimiro, bilopaque, cholimil, ethiodol, diatrast, dionosil, falignost, gadobutrol, gadodiamide, gadopentetate dimeglumine, gastrografin, hexabrix, hippodin, mangafodipir, amidotrizoate, ethiodized oil, imagopaque, iodamide, iodipamide, iodixanol, iodophene, iophendylate, iomeron, iomeprol, iopamidol, iopanoic acid, iopiperidol, iophendylate, iopromide, iopydol, iosimenol, iothalamic acid, iotrolan, ioversol, ioxilan, ioxaglic acid, isopaque, ipodate, meglumine iothalamate, meglumine acetrizoate, meglumine diatrizoate, metrizamide, myelotrast, omnipaque, osbil, optiray, optojod, opacoron, perflutren, phenobutiodil, phentetiothalein sodium, priodax, propyliodone, skiodan, sodium iodomethamate, sodium diatrizoate, telepaque, teridax, tetrabrom, thorotrast, triognost, 1,3,5-Tri-n-hexyl-2,4,6-triiodobenzene, tyropanoate, visipaque or xenetix, and/or a pharmaceutically acceptable salt, and/or a prodrug, and/or a solvate thereof.

Some examples (Schering, Bracco Industria Chimica, Univ. California, Nyegaard, Cook Imaging Corporation, Mallinckrodt, Eprova, Nycomed and Savag¹⁵) of RM suitable for use herein are described within European patent applications EP 0 022 744, EP 0 023 992, EP 0 026 281, EP 0 033 426, EP 0 108 638 and EP 0 317 492, the international applications WO 87/00757 and WO 89/08101, the US patents US 2,776,241, US 3,290,366, US 3,360,436, and US 5,349,085, the British application GB 1 321 591 as well as within the German patents DE 2 547 789, DE 2 726 196 and DE 2 909 439, without limiting the group of RM.

By a "therapeutically effective amount" of a drug or pharmacologically active agent is meant a nontoxic but sufficient amount of the drug or agent to provide the desired effect. In the combination therapy of the present invention, a "therapeutically effective amount" of one component of the combination is the amount of that compound that is effective to provide the desired effect when used in combination with the other components of the combination. The amount that is "effective" will vary from subject to subject, depending on the age and general condition of the individual, the particular active agent or agents, and the like. It thus is not always possible to specify an exact "therapeutically effective amount". However, an appropriate "therapeutically effective amount" in any individual case may be determined by an ordinary skill in the art using routine experimentation.

The at least one RM is not earlier administered until the plasma level of the at least one selective adenosine A1 receptor antagonist has reached a concentration of 10-500 ng/ml. The invention also pertains to any arbitrary concentration or concentration ranges, which lie within the range of 10-500 ng/ml. In a further embodiment, the at least one selective adenosine A1 antagonist has a concentration of 10, 20, 30, 40, 50, 60, 70, 80, 90, 100, 110, 120, 130, 140, 150, 160, 170, 180, 190, 200, 210, 220, 230, 240, 250, 260, 270, 280, 290, 300, 310, 320, 330, 340, 350, 360, 370, 380, 390, 400, 410, 420, 430, 440, 450, 460, 470, 480, 490, and 500 ng/ml, and any arbitrary concentration or concentration ranges, which lie in any ranges defined by two of the before mentioned concentration values, where the lower limit of said range is defined by the minor value and the upper limit of said range by the upper value, e.g. a range of 110-180 ng/ml, 370-390 ng/ml, 10-150 ng/ml, etc. The invention thus pertains to the use comprising the at least one radiocontrast media to be not earlier administered until the therapeutically effective amount of said at least one selective adenosine A1 receptor antagonist is sufficient to provide a plasma level concentration of 10-500 ng/ml, in an embodiment 20-400 ng/ml, in an embodiment 30-300 ng/ml. The invention thus further pertains to a pharmaceutical combination comprising the at least one radiocontrast media to be not earlier administered until the therapeutically effective amount of said at least one selective adenosine A1 receptor antagonist is sufficient to provide a plasma level concentration of 10-500 ng/ml, in an embodiment 20-400 ng/ml, in an embodiment 30-300 ng/ml. The invention further pertains to a use comprising the at least one radiocontrast media to be not earlier administered until the therapeutically effective amount of said at least one selective adenosine A1 receptor antagonist is sufficient to provide a plasma level concentration of 10-500 ng/ml, in an embodiment 20-400 ng/ml, in an embodiment 30-300 ng/ml. The invention further pertains to a pharmaceutical combination comprising the at least one radiocontrast media to be not earlier administered until the therapeutically effective amount of said at least one selective adenosine A1 receptor antagonist is sufficient to provide a plasma level concentration of 10-500 ng/ml, in an embodiment 20-400 ng/ml, in an embodiment 30-300 ng/ml.

The time period of application of the maintenance dosage of the at least one selective A1 adenosine antagonist is sufficient to maintain the plasma level of the at least one selective A1 adenosine on a concentration of 10-500 ng/ml. The invention also pertains to any arbitrary concentration or concentration range, which lie within the range of 10-500 ng/ml. In a further embodiment, the at least one selective adenosine A1 antagonist has a concentration of 10, 20, 30, 40, 50, 60, 70, 80, 90, 100, 110, 120, 130, 140, 150, 160, 170, 180, 190, 200, 210, 220, 230, 240, 250, 260, 270, 280, 290, 300, 310, 320, 330, 340, 350, 360, 370, 380, 390, 400, 410, 420, 430, 440, 450, 460, 470, 480, 490, and 500 ng/ml, and any arbitrary concentration or concentration range, which lie in any ranges defined by two of the before mentioned concentration values, where the lower limit of said range is defined by the minor value and the upper limit of said range by the upper value, e.g. a range of 10-180 ng/ml, 320-390 ng/ml, 100-150 ng/ml, etc.

The time period of application of the maintenance dosage of the at least one selective A1 adenosine antagonist lies between 0.5-6 hours to maintain the plasma level of the at least one selective A1 adenosine on a concentration of 10-500 ng/ml. The invention further pertains to a period of any arbitrary time interval which lies within time period of 0.5-6 hours. In a further embodiment, the time period of administration of the maintenance dosage is 0.1, 0.3, 0.5, 1, 1.5, 2, 2.5, 3, 3.5, 4, 4.5, 5, 5.5 and 6 hours, and any arbitrary period which lies in any ranges defined by two of the before mentioned hour values, where the lower limit of said range is defined by the minor value and the upper limit of said range by the upper value, e.g. a range of 1-2 hours, 2-5 hours, 2.5-3.5 hours, etc.

The at least one selective adenosine A1 receptor antagonist may be administered intravenously in a loading dosage followed by a maintenance dosage, and the amount of the at least one selective adenosine A1 receptor antagonist loading dosage is administered at a time period of 5-25 minutes, prior to the administration of said at least one radiocontrast media, and the maintenance dosage of the at least one selective adenosine A1 receptor antagonist is administered over a period of up to 6 hours subsequent to administration of the loading dosage of said at least one selective A1 receptor antagonist, in a further embodiment over a period of up to 0.5, 1, 1.5, 2, 2.5, 3, 3.5, 4, 4.5, 5, 5.5 and 6 hours. The invention pertains to a period of any arbitrary time interval which lies within time period of 5-25 minutes prior to the administration of said at least one radiocontrast media, and the maintenance dosage of the at least one selective adenosine A1 receptor antagonist is administered over a period of up to 6 hours subsequent to administration of the loading dosage of said at least one selective A1 receptor antagonist, in a further embodiment over a period of up to 0.1, 0.3, 0.5, 1, 1.5, 2, 2.5, 3, 3.5, 4, 4.5, 5, 5.5 and 6 hours. In a further embodiment the at least one selective adenosine A1 receptor antagonist may be administered intravenously at 5, 6, 7, 8, 9, 10, 11, 12, 13, 14, 15, 16, 17, 18, 19, 20, 21, 22, 23, 24 and 25 minutes, and any arbitrary period which lies in any ranges defined by two of the before mentioned minute values, where the lower limit of said range is defined by the minor value and the upper limit of said range by the upper value, e.g. a range of 10-18 minutes, 20-25 minutes, 12-15 minutes, etc., prior to the administration of said at least one radiocontrast media, and the maintenance dosage of the at least one selective adenosine A1 receptor antagonist is administered over a period of up to 6 hours subsequent to administration of the loading dosage of said at least one selective A1 receptor antagonist, in a further embodiment over a period of up to 0.5, 1, 1.5, 2, 2.5, 3, 3.5, 4, 4.5, 5, 5.5 and 6 hours. The invention pertains thus to a use comprising the therapeutically effective amount of said at least one selective adenosine A1 receptor antagonist in a loading dosage to be administered intravenously followed by a maintenance dosage, and the amount of the at least one selective adenosine A1 receptor antagonist loading dosage to be administered at a time period of 5-25 minutes, in an embodiment 10-20 minutes, in an embodiment of 13-17 minutes, in an embodiment 15 minutes prior to the administration of said at least one radiocontrast media, and comprising the maintenance dosage of the at least one selective adenosine A1 receptor antagonist to administered over a period of up to 6 hours subsequent to administration of the loading dosage of said at least one selective A1 receptor antagonist. The invention pertains thus further to a pharmaceutical combination comprising the therapeutically effective amount of said at least one selective adenosine A1 receptor antagonist in a loading dosage to be administered intravenously followed by a maintenance dosage, and the amount of the at least one selective adenosine A1 receptor antagonist loading dosage to be administered at a time period of 5-25 minutes, in an embodiment 10-20 minutes, in an embodiment of 13-17 minutes, in an embodiment 15 minutes prior to the administration of said at least one radiocontrast media, and comprising the maintenance dosage of the at least one selective adenosine A1 receptor antagonist to administered over a period of up to 6 hours subsequent to administration of the loading dosage of said at least one selective A1 receptor antagonist. The invention pertains thus further to a kit comprising the therapeutically effective amount of said at least one selective adenosine A1 receptor antagonist in a loading dosage to be administered intravenously followed by a maintenance dosage, and the amount of the at least one selective adenosine A1 receptor antagonist loading dosage to be administered at a time period of 5-25 minutes, in an embodiment 10-20 minutes, in an embodiment of 13-17 minutes, in an embodiment 15 minutes prior to the administration of said at least one radiocontrast media, and comprising the maintenance dosage of the at least one selective adenosine A1 receptor antagonist to administered over a period of up to 6 hours subsequent to administration of the loading dosage of said at least one selective A1 receptor antagonist.

This invention in its broad scope is not limited to specific dosage forms, carriers, excipients, or the like, as such may vary. It is also to be understood that the terminology used herein is for the purpose of describing particular embodiments only, and is not intended to be limiting.

It must be noted that as used in this specification and the appended claims, the singular forms "a", "an", and "the" include plural referents unless the context clearly dictates otherwise.
Thus, for example, reference to "a therapeutically effective agent" includes a single agent as well as two or more different agents in combination, and reference to "a carrier" includes mixtures of two or more carriers as well as a single carrier, and the like.

The terms "A1AR" "selective adenosine A1 antagonist" and "selective adenosine A1 receptor antagonist" are used interchangeably herein to refer to a chemical compound that induces a desired pharmacological, physiological effect.

The at least one selective adenosine A1 antagonist may be administered orally and/or intravenously. The invention thus pertains to the use comprising the therapeutically effective amount of said at least one selective adenosine A1 receptor antagonist to be administered orally, in an embodiment in an extended release formulation, prior to the administration of the at least one radiocontrast agent.

The invention thus pertains to the use comprising the therapeutically effective amount of said at least one selective adenosine A1 receptor antagonist in a loading dosage to be administered intravenously followed by a maintenance dosage, and the amount of the at least one selective adenosine A1 receptor antagonist loading dosage to be administered at a time period of 5-25 minutes, in an embodiment 10-20 minutes, in an embodiment of 13-17 minutes, in an embodiment 15 minutes prior to the administration of said at least one radiocontrast media, and comprising the maintenance dosage of the at least one selective adenosine A1 receptor antagonist to administered over a period of up to 6 hours subsequent to administration of the loading dosage of said at least one selective A1 receptor antagonist.

The invention thus pertains to a pharmaceutical combination comprising the therapeutically effective amount of said at least one selective adenosine A1 receptor antagonist to be administered orally, in an embodiment in an extended release formulation, prior to the administration of the at least one radiocontrast agent.

The invention thus pertains to a pharmaceutical combination comprising the therapeutically effective amount of said at least one selective adenosine A1 receptor antagonist in a loading dosage to be administered intravenously followed by a maintenance dosage, and the amount of the at least one selective adenosine A1 receptor antagonist loading dosage to be administered at a time period of 5-25 minutes, in an embodiment 10-20 minutes, in an embodiment of 13-17 minutes, in an embodiment 15 minutes prior to the administration of said at least one radiocontrast media, and comprising the maintenance dosage of the at least one selective adenosine A1 receptor antagonist to administered over a period of up to 6 hours subsequent to administration of the loading dosage of said at least one selective A1 receptor antagonist.

The invention thus pertains to a kit comprising the therapeutically effective amount of said at least one selective adenosine A1 receptor antagonist to be administered orally, in an embodiment in an extended release formulation, prior to the administration of the at least one radiocontrast agent.

The invention thus pertains to a kit comprising the therapeutically effective amount of said at least one selective adenosine A1 receptor antagonist in a loading dosage to be administered intravenously followed by a maintenance dosage, and the amount of the at least one selective adenosine A1 receptor antagonist loading dosage to be administered at a time period of 5-25 minutes, in an embodiment 10-20 minutes, in an embodiment of 13-17 minutes, in an embodiment 15 minutes prior to the administration of said at least one radiocontrast media, and comprising the maintenance dosage of the at least one selective adenosine A1 receptor antagonist to administered over a period of up to 6 hours subsequent to administration of the loading dosage of said at least one selective A1 receptor antagonist.

The term "intravenously" is pertaining to parenteral application, which includes injection or infusion into the vein and the artery, without limiting the group of parenteral application forms.

The term "orally" is relating to enteral application, which includes application of e.g. tablets, drops, pills, capsules, pellets, granules, etc. by mouth, without limiting the group of enteral application forms. "Extended release" refers to a pharmaceutically dosage form. The term "extended" includes e.g. "prolonged", "retard", "retentive" and "delayed" dosage forms, without limiting.

The term "container" is referring to a hermetically sealed storage box for pharmaceuticals. It includes storage boxes for fluid pharmaceuticals as e.g. ampoules, vials, flask, dispensers, syringes, etc. as well as storage boxes for solid pharmaceuticals as e.g. blisters, capsules, etc. without limiting the group of storage boxes.

By "pharmaceutically acceptable" such as in the recitation of a "pharmaceutically acceptable carrier", a "pharmaceutically acceptable auxiliary" or a "pharmaceutically acceptable salt" is meant herein a material that is not biologically or otherwise undesirable, i.e., the material may be incorporated into a pharmaceutical combination administered to a patient without causing any undesirable biological effects or interacting in a deleterious manner with any of the other components of the combination in which it is contained. "Pharmacologically active", as in a "pharmacologically active" derivative or metabolite, refers to a derivative or metabolite having the same type of pharmacological activity as the parent compound and approximately equivalent in degree. When the term "pharmaceutically acceptable" is used to refer to a derivative of an active agent, it is to be understood that the compound is pharmacologically active as well, i.e., therapeutically effective for the treatment of radiocontrast media induced nephropathy.

"Carriers "or "pharmaceutically acceptable auxiliary" as used herein refer to conventional pharmaceutical acceptable excipient materials suitable for drug administration, and include any such materials known in the art that are nontoxic and do not interact with other components of a pharmaceutical combination or drug delivery system in a deleterious manner.

As used herein, the terms "comprising" and "including" are used herein in their open, non-limiting sense.

The term "prodrug" as used herein, represents derivatives of the compounds of the invention that are drug precursors which, following administration to a patient, release the drug in vivo via a chemical or physiological process. As used herein, the term "prodrug" includes metabolic precursors. In particular, prodrugs are derivatives of the compounds of the invention in which functional groups carry additional constituents which may be cleaved under physiological conditions in vivo and thereby releasing the active principle of the compound (e. g., a prodrug on being brought to a physiological pH or through an enzyme action is converted to the desired drug form).

The term "pharmaceutically acceptable salts" refers to salt forms that are pharmacologically acceptable and substantially non-toxic to the subject being administered the compounds of the invention.

The term "solvates" pertains to the association of suitable organic solvent molecules with molecules or ions of an A1AR. As used herein, the term "solvates" refers both to stable solvates, containing a defined number of solvent molecules pro molecule of a compound of formula I, and inclusion complexes, which are less stable and contain a variable number of solvent molecules pro molecule of a A1AR.

The term "treatment" as used herein refers to reduction in severity and/or frequency of symptoms, elimination of symptoms and/or underlying cause, prevention of the occurrence of symptoms and/or their underlying cause, and improvement or remediation of damage. Thus, for example, "treatment" of a patient involves prevention of a particular disorder or adverse physiological event in a susceptible individual as well as treatment of a clinically symptomatic individual.

The "increase in serum creatinine level" induced by radiocontrast media might be transient, persistent or irreversible, in an embodiment transient. Reference values for serum creatinine levels (see http://www.rnceus.com/renal/renalcreat.html¹⁶) in adult male lies approximately at 0.8 - 1.4 mg/dl, in adult female at 0.6 - 1.1 mg/dl, and in children at 0.2 - 1.0 mg/dl. An arbitrary range of values of between 25% - 50% or even higher increase in serum creatinine levels from reference values defines CIN. An increase of any arbitrary value within in the range of 25-70% in serum creatinine levels defines CIN. In a further embodiment, an increase of 25, 30, 35, 40, 45, 50, 55, 60, 65 and 70%, and any arbitrary range which lies in any ranges defined by two of the before mentioned values, where the lower limit of said range is defined by the minor value and the upper limit of said range by the upper value, e.g. a range of 25-30%, 25-35%, 30-60%, etc., defines CIN. This definition may in part account for the transient, persistent or irreversible elevations of serum creatinine levels.

The "decrease in renal blood flow" induced by radiocontrast media might be transient, persistent or irreversible, in an embodiment transient. Reference value for blood flow in the kidney is approximately 20% of the cardiac output per minute, thus lies at 1000 ml/min in a healthy human. An arbitrary range of values of between 20% - 80% or even higher decrease in renal blood flow from reference value defines CIN. A decrease of any arbitrary value or value range within in the range of 20-80% in renal blood flow defines CIN. In a further embodiment, a decrease of 20, 25, 30, 35, 40, 45, 50, 55, 60, 65, 70, 75, 80, 85 and 90%, and any arbitrary value or value range which lies in any ranges defined by two of the before mentioned values, where the lower limit of said range is defined by the minor value and the upper limit of said range by the upper value, e.g. a range of 25-30%, 20-35%, 30-60%, etc., defines CIN. This definition may in part account for the transient, persistent or irreversible depressions of renal blood flow values. The renal blood flow can be measured using MRI (magnetic resonance imaging) techniques to determine renal blood flow and renal vascular resistance as well as PAH (para amino hipuric acid) infusion techniques.

Any of the described A1AR may be administered in the form of a salt, ester, amide, prodrug, active metabolite, analog, solvate or the like, provided that the salt, ester, amide, prodrug, active metabolite, analog, or solvate is pharmaceutically acceptable and pharmacologically active in the present context. Salts, esters, amides, prodrugs, metabolites, analogs, solvates and other derivatives of the active agents may be prepared using standard procedures known to those skilled in the art of synthetic organic chemistry and described, for example, by J. March (1992¹⁷).

In a specific embodiment, the invention also relates to a kit comprising in separate containers in a single package pharmaceutical dosage forms for use in combination, comprising, in one separate container a pharmaceutical dosage form comprising at least one A1AR and in a second separate container a pharmaceutical dosage form comprising at least one RM. The kit form is particularly advantageous but not limited to the case when the separate components must be administered in different dosage forms or are administered at different dosage intervals. The selective adenosine A1 dosage forms may favorably be injectable formulations like solutions and suspensions. The kit may further comprise instructions which will typically be written instructions on a package insert, a label, and/or on other components of the kit, and the intravenous dosage forms are as described herein. Each dosage form may be individually housed. The present kits will also typically include means for packaging the individual kit components, i.e., the dosage forms, the container means, and the written instructions for use.

Further on, that the therapeutically effective amount of A1AR is administered in a form, as emphasized above. However, in some cases, a patient may be given each, the therapeutically effective amount of A1AR and the RM, in its own separate dosage form, or a combination of individual "combination" dosage forms containing two or more of the present therapeutically effective A1ARs. When separate dosage forms are used, the A1AR and the RM can be administered at essentially the same time (concurrently), or at separately staggered times (sequentially). Optimum beneficial effects are achieved when the active blood plasma level concentrations of the A1AR agent is maintained while administration of the RM. A form comprising all the A1AR and the RM is, however, much preferred. Such a dosage form provides convenience and simplicity for the patient, thus increasing the chances for patient compliance. Since two or even more active agents are being used together in combination, the potency of each of the agents and the interactive effects achieved by combining them together must also be taken into account. A consideration of these factors is well within the purview of the ordinarily skilled clinician for the purpose of determining the therapeutical effective or prophylactically effective dosage amounts.

The term" irreversible" as used herein can be used interchangeably with the term "permanent".

### STUDY PROTOCOL

### Study 1

Animal studies are performed in 60 anesthetized rats. Renal hemodynamics are assessed, and oxygen tension within the kidney is measured after application of RM. Total blood flow to the kidney is quantified by the transit time method, local hemodynamics by laser-Doppler Flux. In addition, regional oxygen tension of the kidney is assessed and urine is collected to determine urine osmolarity, viscosity and diuresis. Using a recently established technique (Wronski , 2003¹⁸), it is possibly to assess the TGF response in this setting. The RM significantly reduces renal blood flow and perturbs regional kidney oxygenation. This effect is most likely due to viscous properties, as seen by an increase in urine viscosity. These RM effects on renal hemodynamics (renal blood flow and hypoxia) are alleviated or even reversed by prior administration of the A1AR antagonists.

Two protocols are made: Protocol 1: Fluid restriction takes place 24 h before experiments. This leads to augmented concentration of RM in the tubular system. Catheters, transit-time flowmeters, laser-Doppler probes and sounds for assessing absolute pO₂ are implanted. Control measurements are recorded, and then the RM is given. Protocol 2: Measures will then be repeated. In the fluid replete animal, urine volume, osmolarity and viscosity are determined. Control measurements are recorded, then, the RM is given.

Assessment of renal blood flow, oxygen tension and regional blood flows and the TGF response in rats after water restriction take place. Reduced plasma volume is a generally recognized risk factor, since CM is concentrated in the tubules during antidiuresis.

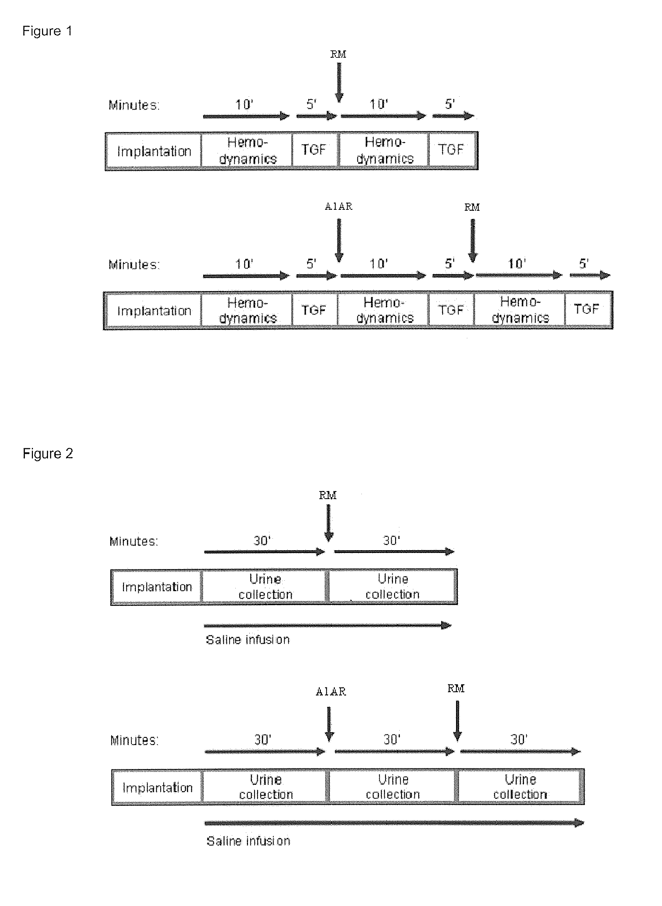

Figure 1 depicts the protocols. In the top panel, the RM is given after control measurements (N=15). The bottom panel dicts the series where the A1AR is given prior to the RM (N=15).

In order to collect sufficient urine, volume repleted rats are used. Diuresis, urine osmolality and viscosity are assessed for control and the RM (N=15, Figure 2 top panel), and for control, the A1AR and the A1AR + the RM (N=15, Figure 2 bottom panel). All experiments are performed on adult, male Wistar rats obtained from the animal facility of the institute. The rats are housed in groups. All animals are randomly distributed to the protocols. The animals are identified by cage number. A standard rat diet (Altromin 1324, Altromin GmbH, D-32791 Lage) serve as chow. Feeding and drinking is discontinued approx. 12 hours before the surgery for protocol 1. In protocol 2, drinking is allowed ad libitum. Drinking water is offered ad libitum, except for a time period of 12h before CM application. Thus, the animals are water deprived. In protocol 2, water is offered ad libitum until immediately before the experiment. Granulated textured wood (Granulat A2, J. Brandenburg, D-49424 Goldenstedt) is used as bedding material for the cages. The cages are changed and cleaned every day between 6:00 and 8:00 a.m. During the acclimatization, the animals are kept in groups of 3 - 5 animals in MAKROLON cages each (type 4). At a room temperature of 22° C ± 3 ° C and a relative humidity of 60% ± 20%. Deviation might be caused for example during the cleaning procedures. Anesthesia is introduced and maintained by urethane. Rats are placed on a heated table to maintain body temperature at 37°C throughout the surgery. The body temperature is controlled during the study. After an incision in the left groin the femoral artery is carefully prepared and cannulated with a polypropylene catheter (PP 10) to measure the renal perfusion pressure (RPP). Another catheter (PP 50) of the same material is placed into the carotid artery to measure systemic blood pressure (BP) and heart rate (HR). Finally, an inflatable cuff is placed around the abdominal aorta; one above and the other below the origin of the renal arteries. A servo controlled inflation of the proximal cuff allowed it to reduce and maintain renal perfusion pressure at a preset level. Two 500µm diameter optical fibers (Moore instruments, GB) are implanted into the cortex and the medulla of left kidney, and an ultrasound transit time flowprobe (1 RB, Transonic Systems inc, USA) is placed around the renal artery of the same kidney to determine local blood flows (LFC and LFM respectively) and total kidney blood flow (RBF). pO₂ is likewise locally determined. Local blood flow is measured and processed by a laser- Doppler flowmeter (Moore Instruments, GB). The arterial catheter is connected to the calibrated pressure transducer. The inflatable cuff is connected to an extracorporal servo control system and the flow probes are connected via extension cables to the Flowmeters. Oxygen partial pressure sensing probes are positioned in a corresponding manner. After analog to digital conversion all data (BP, RPP, RBF, LFC, LFM, local oxygen tension) are stored on-line in ASCII format by a computer system (IBM compatible AT). After implantation and stabilization, the experiment is started. The test solutions are infused. After 5 min equilibrium, measurements of RBF, local fluxes and local pO₂ is commenced. Then, a 5 min step response is obtained to assess TGF. Urine is collected 35 min to evaluate diuresis, osmolality and viscosity. When required, modifications are made to the protocols. Total and regional RBF and oxygen tension in the renal medulla and cortex are assessed according to prior studies (Flemming, 2000 and 2001¹⁹) by measuring laser-Doppler-fluxes and direct assessment of pO₂. After the calculation of individual mean values of every parameter, these mean values of every animal are used to calculate group averages and standard errors of each control/intervention group. The latter are used to test differences for statistical significance, in an embodiment levels of less than 0,05 are considered to indicate significance. The used test methods are chosen with respect to the parameters of the underlying data.

### Study 2

A study analogous to the one described by Yao (2000¹⁰) with selected variations in the protocol is performed. In contrast to the Yao study, chronic as well as acute experiments are carried out. Indometacine is used in addition to L-name (N-ω-nitro-L-arginine methyl ester) in this study.

### BRIEF DESCRIPTION OF THE FIGURES

Figure 1: In volume restricted rats, hemodynamic measurements are made, and the TGF response is assessed
Figure 2: Experimental setting 2 is used for collecting urine. Diuresis, urine osmolality and urine viscosity are determined.

### CITED DOCUMENTS

¹ Andrew E, Berg KJ, "Nephrotoxic effects of X-ray contrast media", J Toxicol Clin Toxicol. 2004;42(3):325-32
² http://www.fpnotebook.com/REN70.htm and http://www.fpnotebook.com/REN38.htm (15-June-2006)
³ Ueda J, Nygren A, Hansell P, Ulfendahl HR. "Effect of intravenous contrast media on proximal and distal tubular hydrostatic pressure in the rat kidney". Acta Radiol 1993; 34(1):83-87
⁴Olivera A, Lamas S, Rodriguez-Puyol D, Lopez-Novoa JM. "Adenosine induces mesangial cell contraction by an A1-type receptor". Kidney Int 1989; 35(6):1300-1305
⁵ Porter, "Contrast-associated nephropathy" Am. J. Cardiol., (1989), 64(9), 22E-26E
⁶ Nikolsky E, Aymong ED, Dangas G, Mehran R. "Radiocontrast nephropathy: identifying the high-risk patient and the implications of exacerbating renal function", Rev Cardiovasc Med 2003; 4 Suppl 1:S7-S14
⁷ a) Shammas et al., "Aminophylline does protect against radiocontrast nephropathy in patients undergoing percutaneous angiographic procedures", J Invasive Cardiol (2001), 13(11), 738-40; b) Welch et al. "Adenosine A1 receptor antagonists in the kidney: effects in fluid-retaining disorders", Curr Opin Pharmacol (2002), 2(2), 165-70; c) Huber et al. "Effect of theophylline on contrast material-nephropathy in patients with chronic renal insufficiency: controlled, randomized, double-blinded study", Radiology (2002), 223(3), 772-9
⁸Erley et.al., "Adenosineantagonist theophylline prevents the reduction of glomerularfiltration rate after contrast media application", Kidney Int. (1994), 45, 1425-31
⁹ K. Akawara et al., "Role of adenosine in the renal responses to contrast medium", Kidney Int. (1996), 49(5), 1199-206
¹⁰ K. Yao et al., "The selective adenosine A1 receptor antagonist KW-3902 prevents radiocontrast media-induced nephropathy in rats with chronic nitric oxide deficiency", Jpn J Pharmacol. (2000), 84(3),347-50
¹¹ Greiner, Dissertation "Prophylaxis of Contrast Induced Nephropathy with Theophylline and Acetylcysteine in ICU-Patients", TU München, 19.10.2005
¹² H. Thomas Lee, Michael Jan, Soo Chan Bae, Jin Deok Joo, Farida R. Goubaeva, Jay Yang, and Mihwa Kim, "A1 adenosine receptor knockout mice are protected against acute radiocontrast nephropathy in vivo", Am J Physiol Renal Physiol 290: F1367-F1375, 2006
¹³ EP 1 386 609, filed by CV Therapeutics
¹⁴ WO 99/31101, filed by Univ. South Florida
¹⁵ EP 0 022 744 filed by Schering, EP 0 023 992 filed by Bracco Industria Chimica, EP 0 026 281 filed by Bracco Industria Chimica, EP 0 033 426 filed by Univ. California, EP 0 108 638 filed by Nyegaard, EP 0 317 492 filed by Schering, WO 87/00757 filed by Cook Imaging Corporation, WO 89/08101 filed by Mallinckrodt, US 2,776,241 filed by Schering, US 3,290,366 filed by Mallinckrodt, US 3,360,436 filed by Eprova, US 5,349,085 filed by Nycomed, GB 1 321 591 filed by Nyegaard, DE 2 547 789 filed by Savag, DE 2 726 196 filed by Nyegaard, DE 2 909 439 filed by Schering
¹⁶ http://www.rnceus.com/renal/renalcreat.html (15-June-2006)
¹⁷ Advanced Organic Chemistry: Reactions, Mechanisms and Structure, 4th Edition, New York:Wiley-Interscience, 1992
¹⁸ Wronski T, Seeliger E, Persson PB, Forner C, Fichtner C, Scheller J et al. The step response: a method to characterize mechanisms of renal blood flow autoregulation. Am J Physiol Renal Physiol 2003; 285(4):F758-F764
¹⁹ a) Flemming B, Arenz N, Seeliger E, Wronski T, Steer K, Persson PB. Time-dependent autoregulation of renal blood flow in conscious rats. J Am Soc Nephrol 2001; 12(11):2253-2262 and b) Flemming B, Seeliger E, Wronski T, Steer K, Arenz N, Persson PB. Oxygen and renal hemodynamics in the conscious rat. J Am Soc Nephrol 2000; 11 (1):18-24

## Claims

1. A use of a therapeutically effective amount of at least one selective adenosine A1 antagonist for the manufacture of a medicament for the prevention of nephropathy induced by at least one radiocontrast media, in mammals or humans.

2. A use of a therapeutically effective amount of at least one selective adenosine A1 antagonist for the manufacture of a medicament for the prevention of increase in serum creatinine levels induced by at least one radiocontrast media, preferably of a transient, persistent or irreversible increase in serum creatinine levels induced by radiocontrast media, in mammals or humans.

3. A use of a therapeutically effective amount of at least one selective adenosine A1 antagonist for the manufacture of a medicament for the prevention of decrease in renal blood flow induced by at least one radiocontrast media, preferably of a transient, persistent or irreversible decrease in renal blood flow induced by radiocontrast media, in mammals or humans.

4. A use of a therapeutically effective amount of at least one selective adenosine A1 antagonist for the manufacture of a medicament for the prevention of a risk or need of dialysis in a human or mammalian patient, preferably of transient, persistent or irreversible dialysis, said patient being subject to receive radiocontrast media.

5. A use according to any of claims 1, 2, 3 or 4, wherein the time period of application of the therapeutically active amount of said at least one selective A1 adenosine antagonist is sufficient to maintain the plasma level of the at least one selective A1 adenosine on a concentration of 10-500 ng/ml, preferably 20-400 ng/ml, more preferably 30-300 ng/ml.

6. A use according to any of claims 1, 2, 3, 4 or 5, comprising the therapeutically effective amount of said at least one selective adenosine A1 receptor antagonist in a loading dosage to be administered intravenously and comprising a maintenance dosage, and the amount of the at least one selective adenosine A1 receptor antagonist loading dosage to be administered at a time period of 5-25 minutes, preferably 10-20 minutes, more preferably of 13-17 minutes, more preferably 15 minutes prior to the administration of said at least one radiocontrast media, and comprising the maintenance dosage of the at least one selective adenosine A1 receptor antagonist to administered over a period of up to 6 hours subsequent to administration of the loading dosage of said at least one selective A1 receptor antagonist.

7. A use according to any of claims 1, 2, 3, 4 or 5, comprising the therapeutically effective amount of said at least one selective adenosine A1 receptor antagonist in an orally, preferably in an extended release formulation, to be administered prior to the administration of the at least one radiocontrast agent.

8. A use according to any of claims 1, 2, 3, 4, 5, 6 or 7, comprising the at least one radiocontrast media to be not earlier administered until the therapeutically effective amount of said at least one selective adenosine A1 receptor antagonist is sufficient to provide a plasma level concentration of 10-500 ng/ml, preferably 20-400 ng/ml, more preferably 30-300 ng/ml.

9. A use according to any of claims 1, 2, 3, 4, 5, 6, 7 or 8, comprising a time period of application of the maintenance dosage of a therapeutically effective amount of said at least one selective A1 adenosine antagonist to be sufficient to maintain the plasma level of the at least one selective A1 adenosine on a concentration of 10-500 ng/ml, preferably 20-400 ng/ml, more preferably 30-300 ng/ml.

10. A use according to any of claims 1, 2, 3, 4, 5, 6, 7, 8 or 9, wherein the therapeutically effective amount of said at least one selective adenosine A1 receptor antagonist is selected from the group of XAC, DPCPX, CPX, NAX, ENX, CVT-124, PACPX, BW-A844U, BW-A 522, SPT, KW-3902, KF15372, KFM 19, MDL 102,503, N-0861, N-0840, FK-352, FK-838, FK-453, WRC-0571, WRC-0342, WRC-0006, WRC-0007, DTI-0017, FR-166124, IRFI-165, GP-04012, EPI-2010, BW-1205U90, 4-[2-phenyl-7H-pyrrolo[2,3-d]-pyrimidin-4-ylamino]-trans-cyclohexanol, 4S-4-hydroxy-1-2-phenyl-7H-pyrrolo[2,3-d]pyrimidin-4-yl-L-prolinamide, N⁶-Butyl-8-phenyladenine, N⁶-Butyl-8-phenyladenine, 8-Phenyltheophylline, theophylline, aminophylline, midaxifylline, apaxifylline, naxifylline, Adentri, and/or a pharmaceutically acceptable salt, and/or a prodrug, and/or a solvate thereof.

11. A use according to any of claims 1, 2, 3, 4, 5, 6, 7, 8, 9 or 10, wherein said at least one radiocontrast media is an iodinated or gadolinium-based radiocontrast media selected from the group consisting of bunaiod, biligram, bilimiro, bilopaque, cholimil, ethiodol, diatrast, dionosil, falignost, gadobutrol, gadodiamide, gadopentetate dimeglumine, gastrografin, hexabrix, hippodin, mangafodipir, amidotrizoate, ethiodized oil, imagopaque, iodamide, iodipamide, iodixanol, iodophene, iophendylate, iomeron, iomeprol, iopamidol, iopanoic acid, iopiperidol, iophendylate, iopromide, iopydol, iosimenol, iothalamic acid, iotrolan, ioversol, ioxilan, ioxaglic acid, isopaque, ipodate, meglumine iothalamate, meglumine acetrizoate, meglumine diatrizoate, metrizamide, myelotrast, omnipaque, osbil, optiray, optojod, opacoron, perflutren, phenobutiodil, phentetiothalein sodium, priodax, propyliodone, skiodan, sodium iodomethamate, sodium diatrizoate, telepaque, teridax, tetrabrom, thorotrast, triognost, 1,3,5-Tri-n-hexyl-2,4,6-triiodobenzene, tyropanoate, visipaque or xenetix, and/or a pharmaceutically acceptable salt, and/or a prodrug, and/or a solvate thereof.

12. A use according to any of claims 1, 2, 3, 4, 5, 6, 7, 8, 9, 10 or 11, wherein the medicament is a fixed combination.

13. A pharmaceutical combination comprising
a) a therapeutically effective amount of at least one selective adenosine A1 antagonist, and
b) at least one radiocontrast media,
wherein the pharmaceutical combination being suitable for simultaneous, separate or step-wise administration to humans or mammals.

14. A pharmaceutical combination according to claim 13, comprising a therapeutically effective amount of said at least one selective adenosine A1 receptor antagonist to be administered intravenously in a loading dosage and comprising a maintenance dosage, and the amount of the at least one selective adenosine A1 receptor antagonist loading dosage to be administered at a time period of 5-25 minutes, preferably 10-20 minutes, more preferably of 13-17 minutes, more preferably 15 minutes prior to the administration of said at least one radiocontrast media, and comprising the maintenance dosage of the at least one selective adenosine A1 receptor antagonist to be administered over a period of up to 6 hours subsequent to administration of the loading dosage of said at least one selective A1 receptor antagonist.

15. A pharmaceutical combination according to claim 13, comprising the therapeutically effective amount of said at least one selective adenosine A1 receptor antagonist in an orally, preferably in an extended release formulation, to be administered prior to the administration of the at least one radiocontrast agent.

16. A pharmaceutical combination according to any of claims 13, 14 or 15, comprising the at least one radiocontrast media to be not earlier administered until the therapeutically effective amount of said at least one selective adenosine A1 receptor antagonist is sufficient to provide a plasma level of a concentration of 10-500 ng/ml, preferably 20-400 ng/ml, more preferably 30-300 ng/ml.

17. A pharmaceutical combination according to any of claims 13, 14, 15 or 16, wherein the time period of application of the at least one selective A1 adenosine antagonist is sufficient to maintain the plasma level of the at least one selective A1 adenosine on a concentration of 10-500 ng/ml, preferably 20-400 ng/ml, more preferably 30-300 ng/ml.

18. A pharmaceutical combination according to any of claims 13, 14, 15, 16 or 17, wherein the therapeutically effective amount of said at least one selective adenosine A1 receptor antagonist is selected from the group of XAC, DPCPX, CPX, NAX, ENX, CVT-124, PACPX, BW-A844U, BW-A 522, SPT, KW-3902, KF15372, KFM 19, MDL 102,503, N-0861, N-0840, FK-352, FK-838, FK-453, WRC-0571, WRC-0342, WRC-0006, WRC-0007, DTI-0017, FR-166124, IRFI-165, GP-04012, EPI-2010, BW-1205U90, 4-[2-phenyl-7H-pyrrolo[2,3-d]-pyrimidin-4-ylamino]-trans-cyclohexanol, 4S-4-hydroxy-1-2-phenyl-7H-pyrrolo[2,3-d]pyrimidin-4-yl-L-prolinamide, N⁶-Butyl-8-phenyladenine, N⁶-Butyl-8-phenyladenine, 8-Phenyltheophylline, theophylline, aminophylline, midaxifylline, apaxifylline, naxifylline, Adentri, and/or a pharmaceutically acceptable salt, and/or a prodrug, and/or a solvate thereof.

19. A pharmaceutical combination according to any of claims 13, 14, 15, 16, 17 or 18, wherein said at least one radiocontrast media is an iodinated or gadolinium-based radiocontrast media selected from the group consisting of bunaiod, biligram, bilimiro, bilopaque, cholimil, ethiodol, diatrast, dionosil, falignost, gadobutrol, gadodiamide, gadopentetate dimeglumine, gastrografin, hexabrix, hippodin, mangafodipir, amidotrizoate, ethiodized oil, imagopaque, iodamide, iodipamide, iodixanol, iodophene, iophendylate, iomeron, iomeprol, iopamidol, iopanoic acid, iopiperidol, iophendylate, iopromide, iopydol, iosimenol, iothalamic acid, iotrolan, ioversol, ioxilan, ioxaglic acid, isopaque, ipodate, meglumine iothalamate, meglumine acetrizoate, meglumine diatrizoate, metrizamide, myelotrast, omnipaque, osbil, optiray, optojod, opacoron, perflutren, phenobutiodil, phentetiothalein sodium, priodax, propyliodone, skiodan, sodium iodomethamate, sodium diatrizoate, telepaque, teridax, tetrabrom, thorotrast, triognost, 1,3,5-Tri-n-hexyl-2,4,6-triiodobenzene, tyropanoate, visipaque or xenetix, and/or a pharmaceutically acceptable salt, and/or a prodrug, and/or a solvate thereof.

20. A kit comprising
a) a therapeutically effective amount of at least one selective adenosine A1 antagonist, and
b) at least one radiocontrast media.
wherein the pharmaceutical combination being suitable for simultaneous, separate or step-wise administration to humans or mammals.

21. A kit according to claim 20, comprising
a) a loading dosage container of the therapeutically effective amount of said at least one selective adenosine A1 receptor antagonist to be administered intravenously
b) a maintenance dosage container of the therapeutically effective amount of said at least one selective adenosine A1 receptor antagonist to be administered intravenously
c) at least one radiocontrast media
comprising the at least one selective adenosine A1 receptor antagonist to be administered intravenously in a loading dosage and comprising a maintenance dosage, and the amount of the at least one selective adenosine A1 receptor antagonist loading dosage to be administered at a time period of 5-25 minutes, preferably 10-20 minutes, more preferably of 13-17 minutes, more preferably 15 minutes prior to the administration of said at least one radiocontrast media, and comprising the maintenance dosage of the at least one selective adenosine A1 receptor antagonist to be administered over a period of up to 6 hours subsequent to administration of the loading dosage of said at least one selective A1 receptor antagonist.

22. A kit according to claim 20 comprising
a) a container with the therapeutically effective amount of said at least one selective adenosine A1 receptor antagonist to be administered orally
b) at least one radiocontrast media
comprising the at least one selective adenosine A1 receptor antagonist in an orally, preferably in an extended release formulation, to be administered prior to the administration of the at least one radiocontrast agent.

23. A kit according to any of claims 20, 21 or 22 comprising the therapeutically effective amount of said at least one radiocontrast media to be not earlier administered until the therapeutically effective amount of said at least one selective adenosine A1 receptor antagonist is sufficient to provide a plasma level of a concentration of 10-500 ng/ml, preferably 20-400 ng/ml, more preferably 30-300 ng/ml.

24. A kit according to any of claims 20, 21, 22 or 23, wherein the time period of application of the therapeutically effective amount of said at least one selective A1 adenosine antagonist is sufficient to maintain the plasma level of the at least one selective A1 adenosine on a concentration of 10-500 ng/ml, preferably 20-400 ng/ml, more preferably 30-300 ng/ml.

25. A kit according to any of claims 20, 21, 22, 23 or 24, wherein the therapeutically effective amount of said at least one selective adenosine A1 receptor antagonist is selected from the group of XAC, DPCPX, CPX, NAX, ENX, CVT-124, PACPX, BW-A844U, BW-A 522, SPT, KW-3902, KF15372, KFM 19, MDL 102,503, N-0861, N-0840, FK-352, FK-838, FK-453, WRC-0571, WRC-0342, WRC-0006, WRC-0007, DTI-0017, FR-166124, IRFI-165, GP-04012, EPI-2010, BW-1205U90, 4-[2-phenyl-7H-pyrrolo[2,3-d]-pyrimidin-4-ylamino]-trans-cyclohexanol, 4S-4-hydroxy-1-2-phenyl-7H-pyrrolo[2,3-d]pyrimidin-4-yl-L-prolinamide, N⁶-Butyl-8-phenyladenine, N⁶-Butyl-8-phenyladenine, 8-Phenyltheophylline, theophylline, aminophylline, midaxifylline, apaxifylline, naxifylline, Adentri, and/or a pharmaceutically acceptable salt, and/or a prodrug, and/or a solvate thereof.

26. A kit according to any of claims 20, 21, 22, 23, 24 or 25, wherein said at least one radiocontrast media is an iodinated or gadolinium-based radiocontrast media selected from the group consisting of bunaiod, biligram, bilimiro, bilopaque, cholimil, ethiodol, diatrast, dionosil, falignost, gadobutrol, gadodiamide, gadopentetate dimeglumine, gastrografin, hexabrix, hippodin, mangafodipir, amidotrizoate, ethiodized oil, imagopaque, iodamide, iodipamide, iodixanol, iodophene, iophendylate, iomeron, iomeprol, iopamidol, iopanoic acid, iopiperidol, iophendylate, iopromide, iopydol, iosimenol, iothalamic acid, iotrolan, ioversol, ioxilan, ioxaglic acid, isopaque, ipodate, meglumine iothalamate, meglumine acetrizoate, meglumine diatrizoate, metrizamide, myelotrast, omnipaque, osbil, optiray, optojod, opacoron, perflutren, phenobutiodil, phentetiothalein sodium, priodax, propyliodone, skiodan, sodium iodomethamate, sodium diatrizoate, telepaque, teridax, tetrabrom, thorotrast, triognost, 1,3,5-Tri-n-hexyl-2,4,6-triiodobenzene, tyropanoate, visipaque or xenetix, and/or a pharmaceutically acceptable salt, and/or a prodrug, and/or a solvate thereof.
